# EUROPÄISCHE PATENTANMELDUNG

(11) **EP 0 848 962 A2**
(43) Veröffentlichungstag der Anmeldung: **24.06.1998**
(21) Anmeldenummer: 97122500.8
(22) Anmeldetag: 19.12.1997
(51) Int. Cl.: A61M 16/00

(54) **Einrichtung zur dosierten Gasversorgung von Anwendern**

(30) Priorität: 21.12.1996 DE 29622321 U
(71) Anmelder: Medicap Medizintechnik GmbH, 35327 Ulrichstein (DE)
(72) Erfinder: Rahn, Günter, 35327 Ulrichstein-Bobenhausen (DE)
(74) Vertreter: Meier, Friedrich, Dipl.-Ing.

(57) **Zusammenfassung**

Die Erfindung bezieht sich auf eine Einrichtung für die dosierte Gasversorgung von Anwendern, insbes. für die Sauerstoffversorgung aus einer Sauerstoffquelle. Zur Steuerung der Dosierung elektrisch Ventil eingesetzt, das von einem beim Einatmen auf Unterdruck ansprechenden Sensor gesteuert wird. Die Öffnungsdauer und damit die Menge des zugeführten Gases wird durch einen Rechner gesteuert. Zumindest die Atemfrequenz, also der Zeitabstand zwischen zwei Atemzügen wird als elektrisches Signale erfaßt und über einen Rechner derart ausgewertet, daß das Ventil hinsichtlich der Öffnungsdauer und gegebenenfalls auch hinsichtlich der Durchlaßmenge pro Zeiteinheit gesteuert wird. Der Rechner kann mit spezifischen Daten des Anwenders programmiert werden.

## Beschreibung

Die Erfindung bezieht sich auf die dosierte Versorgung von Anwendern, insbes. von Patienten mit Atemgas, insbes. mit Sauerstoff. Viele Anwender sind auf die gelegentliche oder auch dauernde Versorgung mit Atemgas, insbesondere mit Sauerstoff angewiesen. Meist geschieht diese Versorgung aus Gasflaschen, die mit entsprechenden Ventilen zur Druckminderung ausgestattet, dem Anwender über Schläuche mit vergleichsweise geringem Querschnitt zugeführt und über sogenannte Nasenbrillen angeschlossen werden. Das meist eingesetzte Gas ist der Sauerstoff. Dieser Sauerstoff ist primär gemeint, wenn nachfolgend das Wort "Gas" zitiert ist.

Bei Anwendungen hat sich gezeigt, daß das Gas nur über einen sehr kurzen Zeitraum zu Beginn der Einatmung, von der Lunge aufgenommen wird. Eine weitere Gaszufuhr während der Einatmung bringt nur sehr wenig. Da das Gas und seine Beschaffung doch einen erheblichen Aufwand erfordert, hat man schon seit Jahren Gassparsysteme eingesetzt, die über einen an die Atemschläuche angeschlossenen Sensor den - beim Einatmen auftretenden - Unterdruck erfassen. Abhängig von diesem Unterdruck wird ein in die Gasversorgung eingeschleiftes elektrisch betätigtes Ventil für eine vorgegebene Zeit geöffnet.

Diese Gassparsysteme sind meist batteriebetriebene Geräte, die von den Anwendern auch außerhalb des Hauses eingesetzt werden können und damit eine gewisse Bewegungsfreiheit gewähren.

Zur Anpassung an den Sauerstoffbedarf können die bekannten Gassparsysteme in Stufen auf entsprechende Sauerstoffmengen, zwischen z.B. 1 l/min bis 8 l/min eingestellt werden. Damit wird schon eine gute Anpassung der Sauerstoffzufuhr an den Leistungsbedarf, z. B. beim Treppensteigen erreicht.

Neben dem Sauerstoffbedarf in Abhängigkeit von Tätigkeiten verschiedener Art, gibt es Bedarfssituationen, die auch von der momentanen Konstitution des Anwenders abhängig ist. In allen Fällen sind manuelle Anpassungen erforderlich, deren Einstellung entsprechende Kenntnisse und eine gute Einschätzung der Situation erfordern.

Der Erfindung liegt die Aufgabe zugrunde, eine Einrichtung der beschriebenen Art zur dosierten, sparsamen Zufuhr von Gas zum Anwender so auszugestalten, daß die Gaszufuhr an jeden Leistungsbedarf ebenso, wie an körperlich bedingte Änderungen hinsichtlich des Sauerstoffbedarfes selbsttätig angepaßt wird.

Die Erfindung macht von einer Einrichtung Gebrauch, bei der die dosierte Gasversorgung von Anwendern, insbes. für die Sauerstoffversorgung aus einer Sauerstoffquelle, z. .B. einer mit Sauerstoff gefüllten Gasflasche, über einen Atemschlauch, unter Verwendung eines in die Versorgungsleitung eingeschleiften elektrisch gesteuerten Ventils, das von einem beim Einatmen auf Druckänderungen, vorzugsweise auf Unterdruck ansprechenden Sensor gesteuert wird, wobei die Öffnungsdauer und damit die Menge des zugeführten Gases entsprechend dem Sauerstoffbedarf einstellbar ist.

Gemäß der Erfindung wird die gestellte Aufgabe , dadurch gelöst, daß die vom Sensor gelieferten Werte zumindest hinsichtlich der Atemfrequenz, also dem Zeitabstand zwischen zwei Atemzügen als elektrische Signale erfaßt, und über einen Rechner derart ausgewertet werden, daß das Ventil von diesem Rechner hinsichtlich der Öffnungsdauer und gegebenenfalls auch hinsichtlich der Durchlaßmenge pro Zeiteinheit gesteuert wird.

Damit die erfaßten, für die Steuerung des Ventils maßgeblichen Werte nicht verfälscht werden, ist es zweckmäßig, mit dem Öffnen des Ventils, die zunächst auftretenden Werte des Druckmeßsensors durch den Rechnern nicht auszuwerten bzw. die Auswertung verzögert vorzunehmen.

Die Meß-, Auswertungs- und Steuerdaten können auch für längere Zeit zwischengespeichert werden, so daß nur bei Änderung der Erfassungsdaten eine Änderung der Steuerdaten ausgelöst wird.

Neben der Erfassungsgröße - Atemfrequenz, also dem Zeitabstand zwischen zwei Atemzügen, kann die Steilheit des Unterdruckanstieges, der über die Auslenkung des Sensors pro Zeiteinheit erfaßt wird, als weiteres Kriterium zur Anpassung der Ventilöffnung eingesetzt und entsprechend ausgewertet werden. Damit kann insbes. bei kurzen Atemzügen das Ventil vergleichsweise lange geöffnet werden.

Auch die Atemtiefe, das heißt die Dauer eines Atemzuges und / oder die Größe des beim Einatmen auftretenden Unterdrucks, kann als Kriterium für die Nachsteuerung der Gaszufuhr ausgewertet werden.

An Hand der Zeichnungen werden wesentliche Merkmale der Erfindung beschrieben und die Wirkungsweise erläutert.

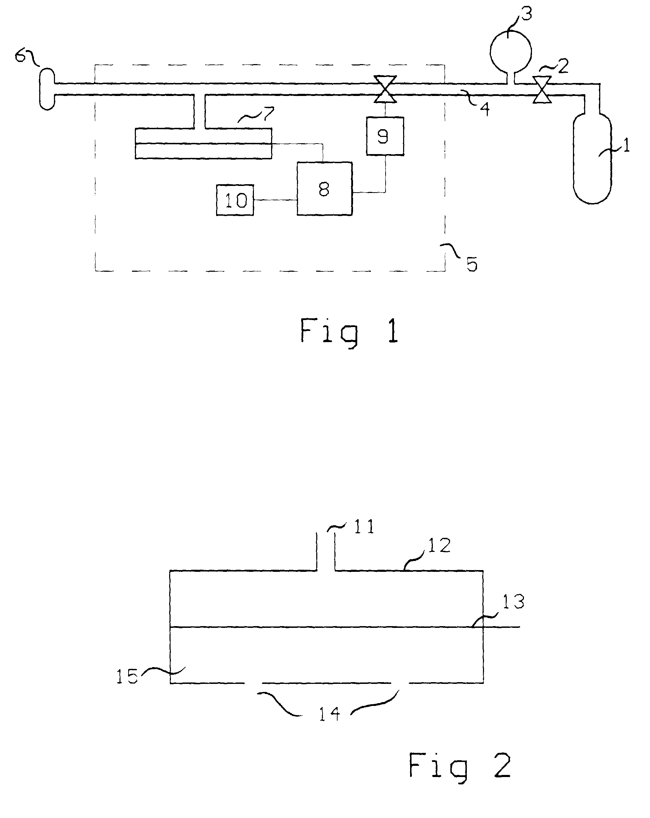

Die Fig. 1 zeigt in schematischer Darstellung den Gesamtaufbau einer Einrichtung zur Versorgung eines Anwenders mit Gas. An eine Gasflasche 1 vorzugsweise eine Sauerstoffflasche, als Beispiel einer Gasversorgung, ist über ein Reduzierventil 2 mit einem Druckmanometer 3, eine Versorgungsleitung 4 angeschlossen. Die Versorgungsleitung 4 ist über eine Einrichtung 5 zur Steuerung der Gaszufuhr zu einem Anwenderanschluß 6, meist eine sogenannte Nasenbrille geführt.

Die Einrichtung 5 besteht aus einer an die Versorgungsleitung 4 angeschlossenen Druckmeßdose 7, einem von der Druckmeßdose 7 gesteuerten Rechner 8, der seinerseits das in die Versorgungsleitung 4 eingeschleifte, elektrische Ventil 9 steuert. Mit dem Rechner 8 ist vorteilhaft eine Programmiereinheit 10 verbunden.

Durch diese Programmiereinheit 10 kann der Rechner 8 an den Bedarf des Anwenders angepaßt werden. Beispielhaft kann die Anpassung an das recht unterschiedliche Atemvolumen von Kindern und Erwachsen erwähnt werden, was zunächst unterschiedliche Daten an der Druckmeßdose 6 ergibt, die vom Rechner 7 aber entsprechend unterschiedlich ausgewertet werden.

Die Fig. 2 zeigt eine Druckmeßdose 7 bekannter Bauart. Über den Anschlußstutzen 11 ist der obere Kammerteil 12, mit der Versorgungsleitung 4 verbunden. Eine dicht mit dem Dosenkörper verbundene und mit einer leitenden Schicht versehene, Membrane 13, trennt den unteren Dosenteil gasdicht ab. Die Membrane 13 bzw. eine aufgebrachte leitende Schicht hat nach außen eine vom Dosengehäuse isolierte, elektrische Verbindung. Die untere Kammerhälfte ist mit einer Druckausgleichsöffnung 14 versehen. Die Druckmeßdose 7 bildet mit der sehr fein auf Druckänderungen ansprechenden Membrane 13 einen feinfühligen Sensor.

Bildet die Membrane 13 zusammen mit dem Gehäuse oder einer nicht gezeichneten Gegenelektrode einen Kondensator, so kann dieser mit dem Anlegen eines Wechselfeldes, durch schon geringe Bewegung der Folie in seinem Übertragungsverhalten verändert werden. Die Änderung des Übertragungsverhaltens wird detektiert. Ein derartiges Wechselfeld kann beispielsweise eine Frequenz von einigen tausend Hertz haben.

An die Stelle einer kapazitiv arbeitenden Druckmeßdose, kann auch eine Druckmeßdose treten, die eine Membrane mit einem z. B. durch sputtern aufgebrachten Widerstand aufweist. Eine durch Dehnung der Membrane bewirke Widerstandsänderung kann als Signalgröße dem Rechner zugeführt werden.

Membranfolien für Druckmeßdosen können aus Polyester mit einer Folienstärke von 12 bis 25 µ hergestellt werden.

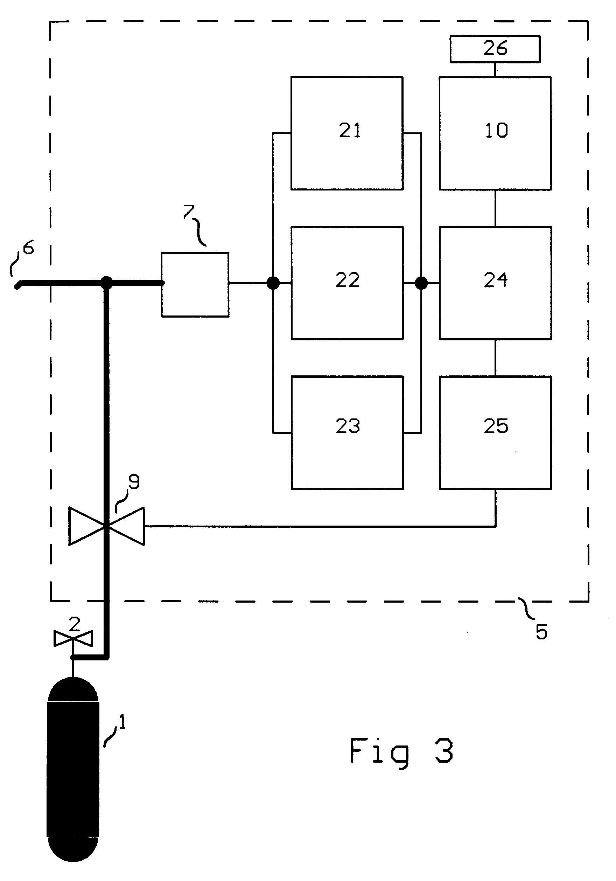

Die Fig. 3 zeigt in schematischer Darstellung die Elemente nach Fig. 1, mit einer symbolischen Darstellung verschiedener Rechnerelemente, durch die eine selbsttätige Anpassung der Gaszufuhr zum Anwender 6 erfolgt. Dabei sind gleiche Teile mit gleichen Bezugszeichen versehen.

Das Rechnerelement 21 ermittelt die Geschwindigkeit der Einatmung, also das Einatemvolumen pro Zeiteinheit aus den Werten der Auslenkung der Membrane. Damit wird einem Luftmangel, dem der Anwender mit heftigen Atemzügen bei der Einatmung begegnet, Rechnung getragen.

Das Rechnerelement 22 erfaßt die Dauer und die Größe des Unterdruckes aus der Membranbewegung. Auch dieses Kriterium kann die Steuereinheit 25 dazu veranlassen, entsprechend dem Resultat der über die Programmiereinheit 10 eingestellten Auswerteeinheit 24, Öffnungsdauer und / oder die Öffnungsweite für das Ventil 2 zu ändern.

Mit dem Rechnerelement 23 wird die Atemfrequenz erfaßt und unter Berücksichtigung der Programmvorgabe das Ventil 9 über die Steuereinheit 25 betätigt.

Vor allem bei kleinen mobilen Einheiten, wird man nicht nur den Sensor und den Rechner, sondern auch das Ventil 9 mit einem Batterieantrieb versehen.

Über eine dem Rechner zugeordnete Anzeige 26, kann auch eine zweckmäßige oder notwendige manuelle Verstellung der Öffnungsweite des Ventils vorgegeben werden. Hierdurch kann eine elektrische Verstellung und damit ein die Batterien stark belastender Energiebedarf eingespart werden, zumal eine Nachstellung der Öffnungsweite der Ventile über eine Durchlaßblende oder dergleichen, meist eine Dauereinstellung ist, die vom Rechner vorgegeben, oder von diesem berücksichtigt werden kann.

Die Programmiereinheit 10 kann auch relevante Kenndaten eines Anwenders aufnehmen und bestimmte, sogar individuell vorgebbare Grenzdaten neben den bekannten Signalen für Gefahren, wie Atemausfall und dergleichen berücksichtigen.

## Patentansprüche

1. Einrichtung für die dosierte Gasversorgung von Anwendern, insbes. für die Sauerstoffversorgung aus einer Sauerstoffquelle, z..B. einer mit Sauerstoff gefüllten Gasflasche, über einen Atemschlauch, unter Verwendung eines in die Versorgungsleitung eingeschleiften elektrisch betätigbaren Ventils, das von einem beim Einatmen auf Unterdruck ansprechenden Sensor gesteuert wird, wobei die Öffnungsdauer und damit die Menge des zugeführten Gases entsprechend dem Sauerstoffbedarf einstellbar ist , **dadurch gekennzeichnet, daß** die vom Sensor gelieferten Werte zumindest hinsichtlich der Atemfrequenz, also dem Zeitabstand zwischen zwei Atemzügen als elektrische Signale erfaßt, und über einen Rechner derart ausgewertet werden, daß das Ventil von diesem Rechner hinsichtlich der Öffnungsdauer und gegebenenfalls auch hinsichtlich der Durchlaßmenge pro Zeiteinheit gesteuert wird.

2. Einrichtung nach Anspruch 1, **dadurch gekennzeichnet, daß** der Rechner mit dem Ansteuern des Ventils, die Sensordaten verzögert, das heißt, zumindest kurzzeitig nicht auswertet.

3. Einrichtung nach Anspruch 1 und 2, **dadurch gekennzeichnet, daß** der Rechner das Ventil zur Anpassung an den Sauerstoffbedarf entsprechend länger oder kürzer öffnet.

4. Einrichtung nach Anspruch 1 bis 3, **dadurch gekennzeichnet, daß** der Rechner die Steilheit des Unterdruckanstieges über die Auslenkung des Sensors pro Zeiteinheit erfaßt, und damit insbes. bei kurzen Atemzügen das Ventil vergleichsweise lange öffnet.

5. Einrichtung nach Anspruch 1 bis 4, **dadurch gekennzeichnet, daß** der Rechner die Atemtiefe über die Dauer und / oder Größe des beim Einatmen auftretenden Unterdrucks erfaßt.

6. Einrichtung nach Anspruch 1 bis 5, **dadurch gekennzeichnet, daß** der Sensor als Druckmeßdose mit einer kapazitiv gegen das Gehäuse wirkenden Membrane ausgebildet und die druckabhängigen Kapazitätsänderungen als Änderung des elektrischen Übertragungsverhaltens erfaßt wird.

7. Einrichtung nach Anspruch 1 bis 6, **dadurch gekennzeichnet, daß** die Membrane des Sensors mit an sich bekannten, insbes. gesputterten Printwiderständen versehen ist, deren Widerstandsänderung nach Größe, Dauer und Änderung pro Zeiteinheit erfaßt wird.

8. Einrichtung nach Anspruch 1 bis 7, **dadurch gekennzeichnet, daß** der Rechner auf individuelle Anwenderdaten, insbes. auf unterschiedliche Atemvolumen von Erwachsenen und Kindern programmierbar ist.
